# EUROPÄISCHE PATENTSCHRIFT

(11) **EP 2 816 328 B1**
(45) Veröffentlichungstag und Bekanntmachung des Hinweises auf die Patenterteilung: **13.01.2021**
(21) Anmeldenummer: 14001946.4
(22) Anmeldetag: 05.06.2014
(51) Int. Cl.: G01F 1/696, G01N 33/00, G01N 27/18

(54) **Verfahren und Durchflussmessgerät zum Bestimmen wenigstens eines Gasparameters eines strömenden Gases**
Method and flow meter for determining at least one gas parameter of a flowing gas
Procédé et débitmètre de détermination d'au moins un paramètre gazeux d'un gaz s'écoulant

(30) Priorität: 20.06.2013 DE 102013010340; 23.01.2014 DE 102014000939
(43) Veröffentlichungstag der Anmeldung: 24.12.2014
(73) Patentinhaber: Diehl Metering GmbH, 91522 Ansbach (DE)
(72) Erfinder: Schirm, Christian, 81543 München (DE)
(74) Vertreter: Diehl Patentabteilung

(56) Entgegenhaltungen:
- EP-A1- 1 391 703
- EP-A1- 1 965 179
- DE-A1-102007 050 792

## Beschreibung

Die Erfindung betrifft ein Verfahren zum Bestimmen wenigstens eines Gasparameters, insbesondere einer Durchflussmenge, eines strömenden Gases, mittels eines Durchflussmessgeräts, umfassend eine Messstrecke mit einem Heizelement und wenigstens drei Temperatursensoren, über die das Gas geführt wird, wobei mindestens ein erster Temperatursensor stromaufwärts von dem Heizelement, mindestens ein zweiter Temperatursensor im Bereich des Heizelements und mindestens ein dritter Temperatursensor stromabwärts von dem Heizelement angeordnet sind, wobei insbesondere das Heizelement selbst als Temperatursensor genutzt werden kann.

Eine Methode zur Messung der Durchflussmenge sowie weiterer Parameter eines durch eine Messstrecke fließenden Gases ist es, das Gas in einem Bereich der Messstrecke zu beheizen und dann beispielsweise die Durchflussmenge des Gases anhand von Temperaturdaten eines stromaufwärts und eines strom- abwärts angeordneten Temperatursensors zu bestimmen. Erfolgt kein Gasfluss und sind die stromaufwärts und stromabwärts angeordneten Temperatursensoren gleich weit von dem Heizelement entfernt, so wird an beiden Temperatursensoren die gleiche Temperatur gemessen. Mit zunehmendem Gasfluss über die Temperatursensoren und das Heizelement erfolgt ein gerichteter Transport des erwärmten Gases vom Heizelement in Richtung des stromabwärts gelegenen Temperatursensors. Damit führ ein zunehmender Gasfluss zu einer zunehmenden Asymmetrie der Temperaturverteilung und damit zu einem zunehmenden Temperaturunterschied, der mit den beiden Temperatursensoren gemessen wird.

Aus der DE 10 2007 050 792 A1 ist ein Strömungssensor mit drei im Strömungsweg hintereinander angeordneten, elektrisch parallel geschalteten temperatursensitiven Widerständen bekannt. Der in der Mitte angeordnete Widerstand kann einerseits als Heizwiderstand dienen (im Messbetrieb), andererseits aber auch als Temperatursensor zur Abklärung des Vorliegens einer eventuellen Sensordrift (im Testmodus).

Die EP 1 965 179 A1 offenbart einen thermischen Durchflusssensor mit Selbstkontrolle, welcher zwei symmetrisch zu einem Heizelement, jedoch in unterschiedlichen Abständen zu diesem, angeordnete Temperatursensorpaare aufweist. Die vom Heizelement weiter entfernten Sensoren dienen dabei der Durchflussmessung, während die näher am Heizelement angeordneten Sensoren der Erzeugung eines Testsignals dienen.

Der gemessene Temperaturunterschied hängt jedoch nicht ausschließlich von der Gasflussmenge ab, sondern auch von weiteren Parametern wie der Umgebungstemperatur, die die Mediumstemperatur und damit den Temperaturbezugspunkt bestimmt, und den Parametern des die Messstrecke durchströmenden Gases bzw. der Zusammensetzung einer die Messstrecke durchströmenden Gasmischung. Mit der beschriebenen Art zur Erkennung eines Durchflusses können also nur dann quantitative Ergebnisse mit hoher Genauigkeit gewonnen werden, wenn zumindest die Umgebungstemperatur und die Art bzw. die Zusammensetzung des Gases bekannt ist.

Insbesondere bei der Messung des Durchflusses von Erdgasen kann sich jedoch die Zusammensetzung des den Durchflussmesser durchströmenden Gases ändern. In diesem Fall ist es notwendig, die gewonnen Daten je nach Art des Gases bzw. der Gaszusammensetzung zu korrigieren. Hierzu müssen weitere Parameter des die Messstrecke durchströmenden Gases ermittelt werden.

In der EP 1 391 703 A1 ist ein thermisches Gasdurchfluss-Messgerät mit Gasqualitätsindikator beschrieben, welches jeweils einen Temperatursensor vor und nach einem Heizelement aufweist und neben der Ermittlung der Durchflussmenge eine Differenzierung zwischen brennbarem und nicht brennbarem Gasgemisch erlaubt.

Zur Ermittlung weiterer Gasparameter können auf jeder Seite des Heizelements wenigstens zwei Temperatursensoren genutzt und damit zusätzliche Informationen über die Temperaturverteilung gewonnen werden. Alternativ oder ergänzend kann auch der Temperaturwert wenigstens eines Temperatursensors unter zwei verschiedenen Bedingungen, beispielsweise einmal unter Gasfluss und einmal bei stehendem Gas gemessen werden oder ähnliches. Dadurch ist es möglich, zusätzlich die Wärmeleitfähigkeit eines Gases zu ermitteln, wodurch für gewisse Gruppen von Gasen eine Kompensation der Durchflussmessung möglich ist.

Die beschriebenen Verfahren können jedoch nur einen zusätzlichen Gasparameter ermitteln, wodurch weiterhin nur Kalibrierungen für wenige Gasgemische möglich sind.

Der Erfindung liegt damit die Aufgabe zugrunde, ein Verfahren zum Bestimmen wenigstens eines Gasparameters anzugeben, das die Nutzung relativ einfacher Sensoren erlaubt, und das zudem eine genaue Bestimmung zusätzlicher Gasparameter für eine Vielzahl von Gasarten erlaubt.

Die Aufgabe wird erfindungsgemäß mit einem Verfahren der eingangs genannten Art dadurch gelöst, dass eine Recheneinrichtung den wenigstens einen Gasparameter in Abhängigkeit der Temperaturmesswerte am ersten und am zweiten und am dritten Temperatursensor bestimmt, und/oder dass die Recheneinrichtung wenigstens zwei separate Gasparameter in Abhängigkeit der Temperaturmesswerte einzelner unterschiedlicher Temperatursensoren und/oder der Kombinationen von Temperaturmesswerten unterschiedlicher Temperatursensoren bestimmt, wobei im Rahmen der Bestimmung der Gasparameter die Temperaturmesswerte am ersten und am zweiten und am dritten Temperatursensor verwendet werden.

Der Erfindung liegt die Idee zugrunde, dass zusätzlich zu den stromaufwärts und stromabwärts des Heizelementes gelegenen Temperatursensoren wenigstens ein zweiter Temperatursensor am Heizelement genutzt wird. Es ist bekannt, einen Temperatursensor am Heizelement ; zu Diagnosezwecken des Durchflussmessgerätes zu nutzen.

Im erfindungsgemäßen Verfahren soll hingegen der Temperaturwert am Temperatursensor im Bereich des Heizelements bei der Ermittlung wenigstens eines Gasparameters genutzt werden, wobei insbesondere das Heizelement selbst als zweiter Temperatursensor genutzt werden kann. Es werden im erfindungsgemäßen Verfahren also die Temperaturwerte von drei Temperatursensoren ermittelt und es werden im Rahmen der Ermittlung von einem oder mehreren Gasparametern alle drei dieser Temperaturwerte genutzt.

Im einfachsten Fall können die Temperaturwerte aller drei Temperatursensoren genutzt werden, um einen einzelnen Gasparameter zu ermitteln. Dies ist beispielsweise dadurch möglich, dass in Vorbereitungsmessungen eine dreidimensionale Wertetabelle erstellt wird, die Kalibrierwerte umfasst, die den Gasparameter in Abhängigkeit von allen drei Temperaturmesswerten angeben.

Beispielsweise kann durch eine solche Wertetabelle eine Durchflussmenge bestimmt werden, die von allen drei Temperaturwerten abhängig ist. Selbstverständlich ist es auch möglich, mit mehreren solcher Wertetabellen mehrere Parameter aus den drei Temperaturmesswerten zu ermitteln, beispielsweise kann auch eine Wärme- oder eine Temperaturleitfähigkeit ermittelt werden. Es ist auch möglich, dass solche Tabellen Informationen über Gaszusammensetzungen oder Gasarten enthalten.

Statt Wertetabellen zu nutzen, ist es selbstverständlich auch möglich, die Abhängigkeit des Gasparameters von den Temperaturwerten beispielsweise durch eine gewichtete Summe der Temperaturwerte der Temperatursensoren auszudrücken. Eine solche gewichtete Summe kann als eine lineare Näherung der dreidimensionalen Abhängigkeit eines Gasparameters angesehen werden. Es ist zudem auch möglich, weitere Summenglieder hinzuzufügen, die beispielsweise vom Produkt zweier Temperaturmesswerte, einer Potenz eines Temperaturmesswerts oder ähnlichem abhängen. Ein großer Vorteil der Nutzung von gewichteten Summen gegenüber der Nutzung von mehrdimensionalen Wertetabellen ist es, dass der Speicherverbrauch in der Recheneinrichtung des Durchflussmessgerätes wesentlich geringer ist, was vorteilhaft ist, da für Durchflussmessgeräte möglichst einfache Recheneinrichtungen genutzt werden sollen.

Es ist dabei auch möglich, dass der Recheneinrichtung bereits vorverarbeitete Temperaturwerte zur Verfügung gestellt werden. So kann beispielsweise eine elektronische Schaltungseinheit vorgesehen sein, die die Temperatursensoren derart verschaltet, dass ein Ausgangssignal der Schaltungseinheit von Temperaturmesswerten mehrerer Temperatursensoren abhängig ist. Dabei können beispielsweise einfache analoge Additions- oder Subtraktionsschaltungen genutzt werden, um lineare Verknüpfungen der Temperaturmesswerte bzw. der Ausgangssignale mehrerer Temperatursensoren bereits vorverarbeitet bereitzustellen.

Das Ermitteln der zuvor erwähnten dreidimensionalen Kalibriertabelle zur Bestimmung eines Gasparameters oder das Finden einer Formel, die eine Gruppe von Gasen gut beschreibt, kann sehr aufwendig sein. Daher ist es auch möglich, dass die Recheneinrichtung wenigstens zwei separate Gasparameter bestimmt. So kann beispielsweise zusätzlich zu der Durchflussmenge eine Wärme- und/oder Temperaturleitfähigkeit des Gases ermittelt werden. Dies kann besonders einfach möglich sein, wenn bestimmte Gasparameter bestimmten unabhängigen Verknüpfungen der Temperaturmesswerte an den Temperatursensoren zugeordnet werden können. In diesem Fall kann beispielsweise eine Summe oder eine Differenz zwischen zwei Temperaturmesswerten genutzt werden, um einen Wert in einer eindimensionalen Kalibriertabelle zu indizieren, der dann, insbesondere interpoliert, ausgelesen wird. Da in diesem Fall nur noch eindimensionale Kalibriertabellen vorliegen, ist eine Kalibrierung eines Durchflussmessgerätes wesentlich einfacher möglich.

Üblicherweise wird wenigstens einer der Gasparameter von wenigstens zwei der Temperaturmesswerte abhängen. Da drei Messwerte dreier Temperatursensoren vorliegen, können drei unabhängige physikalische Parameter des Gases ermittelt werden. Dies können beispielsweise der Gasfluss, die Temperaturleitfähigkeit und die Wärmeleitfähigkeit sein. Die Ermittlung dieser Parameter soll im Folgenden beispielhaft erläutert werden.

Die Durchflussmenge kann wie eingangs erläutert aus der Asymmetrie der Temperaturverteilung an den drei Temperatursensoren bestimmt werden. Eine solche Asymmetrie kann beispielsweise ausgedrückt werden, indem als Messwert für den Gasfluss die Differenz der Temperatur des ersten und des dritten Temperatursensors ermittelt wird. Die Ermittlung einer solchen Differenz kann dadurch erfolgen, dass zwei getrennte Temperaturmesswerte aufgenommen werden und anschließend ein Subtrahieren der Temperaturwerte in der Recheneinrichtung erfolgt.

Mit einem zunehmend größeren Gasfluss wird zunehmend mehr erwärmtes Gas vom Heizelement zum dritten Temperatursensor transportiert, wodurch der Temperaturmesswert am dritten Temperatursensor steigt, wobei die Temperatur aber mit weiter steigendem Durchfluss wieder sinkt. Es wird aufgrund des entgegengerichteten Gasstroms zunehmend schwerer für das Gas von dem Heizelement zum ersten Temperatursensor hin zu diffundieren. Damit bildet der Gasfluss eine monotone Funktion, insbesondere eine Wurzel-Funktion, des Temperaturunterschieds zwischen der Temperatur am dritten Temperatursensor und der Temperatur am zweiten Temperatursensor. Um Nichtlinearitäten dieser Abhängigkeit sowie die Abhängigkeit von weiteren Parametern zu berücksichtigen, kann beispielsweise eine Kalibriertabelle verwendet werden.

Die Temperaturleitfähigkeit des Gases kann beispielsweise aus der Breite der Temperaturverteilung extrahiert werden. Ist die Temperaturleitfähigkeit des Gases hoch, so wird weiter entfernt vom Heizelement noch eine erhöhte Temperatur gemessen. Diese Verbreiterung der Kurve kann beispielsweise ermittelt werden, indem die Summe der Temperaturmesswerte am ersten Temperaturmesssensor und am dritten Temperaturmesssensor betrachtet wird. Ist die Summe dieser beiden Temperaturmesswerte groß, so ist die Temperaturverteilung breit, was wiederum einer großen Temperaturleitfähigkeit entspricht. Wie bereits bei der Ermittlung des Gasflusses aus der Asymmetrie kann auch zur Ermittlung der Temperaturleitfähigkeit aus der Breite der Kurve eine Wertetabelle mit Kalibrierwerten genutzt werden.

Als letzter Gasparameter kann beispielsweise die Wärmeleitfähigkeit des die Messstrecke durchströmenden Gases ermittelt werden. Im erfindungsgemäßen Verfahren erfolgt üblicherweise eine ungeregelte Beheizung des Heizelements. Ist nun die Wärmeleitfähigkeit des durch die Messstrecke strömenden Gases groß, so bedeutet dies, dass eine große Energiemenge vom Heizelement abtransportiert wird. Damit sinkt die Temperatur am Heizelement, wenn dieses mit gleicher Heizleistung beheizt wird. Eine hohe Temperatur am zweiten Temperatursensor entspricht also einer geringen Wärmeleitfähigkeit, eine niedrige Temperatur einer großen Wärmeleitfähigkeit. In diesem Fall kann also der Temperaturwert am zweiten Temperatursensor direkt als der relevante Wert zur Ermittlung der Wärmeleitfähigkeit genutzt werden. Wie bereits zur Bestimmung des Gasflusses und der Temperaturleitfähigkeit kann die Bestimmung des Parameters hier durch Nutzung einer Wertetabelle mit Kalibrierwerten erfolgen.

Wie eingangs erwähnt, erlaubt die Nutzung von drei Temperatursensoren die Ermittlung von drei unabhängigen Gasparametern. Dass bei der vorangehend beschriebenen Ermittlung der Gasparameter, die Gasparameter unabhängig sind, ist leicht daran zu erkennen, dass die jeweiligen charakteristischen Größen, also die Differenz der Temperaturwerte am ersten und am dritten Temperatursensor, die Summe der Temperaturwerte am ersten und am dritten Temperatursensor und die Temperatur am zweiten Temperatursensor linear unabhängige Linearkombinationen der Temperaturwerte an den Temperatursensoren sind. Dies bedeutet, dass auch die entsprechenden charakteristischen Messgrößen linear unabhängig sind. Eine Änderung einer der Größen führt damit nicht automatisch zu einer Änderung einer anderen der Größen. Damit sind die Größen unabhängig.

Häufig ist in einem Durchflussmessgerät ein weiterer Temperatursensor vorgesehen, der die Umgebungstemperatur bzw. die Temperatur des Gases, das weit von dem Heizelement entfernt ist, misst. Dieser wurde in der bisherigen Erläuterung nicht erwähnt, da die Umgebungs- bzw. Gastemperatur direkt als ein unabhängiger Parameter ermittelt werden kann.

Bei der Ermittlung von mehreren Gasparametern ist es insbesondere vorteilhaft, dass die Werte eines Gasparameters in Abhängigkeit wenigstens eines weiteren Parameters korrigiert werden können. Wie erläutert, ist der Gasfluss von der Asymmetrie der Temperaturverteilung abhängig und zusätzlich von der Temperaturleitfähigkeit und der Wärmeleitfähigkeit. Da im erfindungsgemäßen Verfahren jedoch diese weiteren Größen gemessen werden können, ist damit auch eine Korrektur des zunächst ermittelten Gasflusses möglich. Eine solche Korrektur kann nur in eine Richtung erfolgen, das heißt, dass beispielsweise ein ermittelter Gasfluss in Abhängigkeit einer ermittelten Temperaturleitfähigkeit und/oder Wärmeleitfähigkeit korrigiert wird, die Korrektur kann jedoch auch selbstkonsistent erfolgen, das heißt, dass zunächst der Gasfluss durch einen oder beide der anderen Parameter korrigiert wird und anschließend die weiteren Parameter durch den Gasfluss usw.

Wie bereits beispielhaft dargestellt ist es möglich, dass in Abhängigkeit von den Temperaturmesswerten der wenigstens drei Temperatursensoren drei unabhängige Gasparameter bestimmt werden, die durch die Recheneinrichtung in Abhängigkeit dreier linear unabhängiger Linearkombinationen der Temperaturmesswerte des ersten, des zweiten und des dritten Temperatursensors bestimmt werden. Eine solche Linearkombination wird typischerweise als gewichtete Summe berechnet, wobei die Gewichtungen auch negativ sein können. Dies ist insbesondere dann vorteilhaft, wenn bei der Gewichtung, bei der Berechnung wenigstens eines, insbesondere wenigstens zweier Parameter, das Gewicht zweier Temperaturmesswerte ungleich Null ist.

Zudem kann durch wenigstens einen vierten Temperatursensor die Umgebungstemperatur und/oder die Gastemperatur außerhalb des Messbereichs der wenigstens drei Temperatursensoren und beabstandet vom Heizelement, insbesondere vor Eintritt des Gases in den Messbereich des ersten Temperatursensors und/oder nach Austritt aus dem Messbereich des dritten Temperatursensors, gemessen werden. Damit ist es möglich, die Temperatur der Umgebung bzw. des Gases entfernt vom Heizelement bei der Berechnung der Gasparameter zu berücksichtigen. Insbesondere kann diese Temperatur als ein unabhängiger Parameter aufgefasst werden, sie kann aber auch mit den Temperaturwerten der drei Temperatursensoren verknüpft werden.

Es ist zudem möglich, dass bei der Bestimmung wenigstens zweier Gasparameter die Gasparameter wiederholt bestimmt werden, wobei für wenigstens einen der Gasparameter die Bestimmung mit einem anderen zeitlichen Abstand wiederholt wird, als für wenigstens einen anderen der Gasparameter. Häufig ist es wünschenswert, dass ein Durchflussmesser möglichst sparsam arbeitet. Daher sollen unnötige Mess- und Rechenvorgänge vermieden werden. Ist nun zu erwarten, dass sich einer der Gasparameter, beispielsweise eine Zusammensetzung des Gases, sehr viel langsamer ändert als ein weiterer der Gasparameter, beispielsweise die Durchflussmenge, so kann der schneller veränderliche Parameter mit geringfügigem zeitlichen Abstand, beispielsweise jede Sekunde oder mehrmals pro Sekunde, gemessen werden, der langsam veränderliche Parameter jedoch im Abstand von mehreren Sekunden oder gar Minuten. Da für den langsam veränderlichen Parameter keine oder nur geringfügige Veränderungen zwischen den Messungen des schnell veränderlichen Parameters zu erwarten sind, kann der vorangehend gemessene Messwert des langsam veränderlichen Gasparameters dennoch weiterhin zur Korrektur der ermittelten Werte des schneller veränderlichen Gasparameters genutzt werden.

Häufig soll als Gasparameter des Durchflussmessers eine Durchflussmenge ermittelt werden. Dabei kann als einer der Gasparameter eine unkorrigierte Durchflussmenge aus Temperaturmesswerten am ersten und am dritten Temperatursensor bestimmt werden, wobei aus der unkorrigierten Durchflussmenge und wenigstens einem weiteren der ermittelten Gasparameter eine korrigierte Durchflussmenge bestimmt wird.

Insbesondere kann wenigstens einer der ermittelten Gasparameter eine Wärmeleitfähigkeit des Gases oder eine Temperaturleitfähigkeit des Gases beschreiben. Wie eingangs erläutert, kann eine Wärmeleitfähigkeit beispielsweise in Abhängigkeit des Temperaturmesswertes des zweiten Temperatursensors ermittelt werden und eine Temperaturleitfähigkeit in Abhängigkeit der Summe der Messwerte des ersten und des dritten Temperaturmesssensors bzw. aus den einzelnen Werten dieser Temperaturmesssensoren.

Häufig gibt es bei Gasen eine Wechselwirkung zwischen mehreren der gemessenen Gasparameter. So können beispielsweise eine Wärme- und/oder eine Temperaturleitfähigkeit einen Einfluss auf das Verhältnis von Symmetrie der Temperaturverteilung und der Durchflussmenge haben. Im einfachsten Fall kann dies korrigiert werden, indem ermittelte Werte weiterer Gasparameter bei der Ermittlung eines Gasparameters berücksichtigt werden. So kann beispielsweise zwischen mehreren Wertetabellen mit Kalibrierwerten gewechselt und/oder interpoliert werden, es können jedoch auch einfache additive Korrekturfaktoren oder ähnliches genutzt werden.

Es ist möglich, dass als einer der Gasparameter ein korrigierter eine Wärmeleitfähigkeit beschreibender Gasparameter in Abhängigkeit eines unkorrigierten eine Wärmleitfähigkeit beschreibenden Gasparameters und eines eine Durchflussmenge beschreibenden Gasparamteters bestimmt wird. Durch diese Kompensation kann ein Einfluss der Durchflussmenge und damit auch der mittleren Strömungsgeschwindigkeit auf die ermittelte Wärmeleitfähigkeit korrigiert werden.

Eine Korrektur eines Gasparameters oder eine Bestimmung eines Gasparamters ist dabei selbstverständlich auch in Abhängigkeit mehrerer zuvor bestimmter Gasparameter oder jener Temperaturmesswerte, aus denen diese Gasparameter bestimmt sind, möglich. Dabei können die zur Bestimmung bzw. zur Korrektur genutzten Gasparameter bereits vorangehend bestimmt sein, wobei insbesondere zur Bestimmung dieser Gasparameter zu einem anderen Zeitpunkt ermittelte Temperaturmesswerte genutzt werden können, als zur Ermittlung des zu bestimmenden bzw. zu korrigierenden Gasparameters. Alternativ ist es jedoch auch möglich, die Messwerte der wenigstens drei Temperatursensoren zu genau einem Zeitpunkt sowohl zur Bestimmung jener Gasparameter, die der Korrektur bzw. Bestimmung dienen, als auch des Gasparameters, der korrigiert bzw. bestimmt wird, zu nutzen.

Dabei ist es insbesondere möglich, dass als einer der Gasparameter ein eine Temperaturleitfähigkeit beschreibender Gasparameter in Abhängigkeit eines eine Wärmeleitfähigkeit beschreibenden Gasparamters und eines eine Durchflussmenge beschreibenden Gasparameters, die jeweils in Abhängigkeit wenigstens eines momentanen oder vorab erfassten Temperaturmesswertes bestimmt werden, bestimmt wird.

Da eine Abhängigkeit zwischen Gasparametern häufig beidseitig ist, das heißt, dass beispielsweise eine ermittelte Durchflussmenge von der tatsächlichen Temperaturleitfähigkeit abhängig ist und eine ermittelte Temperaturleitfähigkeit von der tatsächlichen Durchflussmenge, kann eine Korrektur der Werte durch ein iteratives Verfahren erfolgen. Daher ist es möglich, dass wenigstens zwei der Gasparameter in einem iterativen Verfahren ermittelt werden, wobei abwechselnd in einem ersten Schritt wenigstens ein erster der Gasparameter in Abhängigkeit wenigstens eines zweiten der Gasparameters, sowie insbesondere des Temperaturmesswerts an wenigstens einem der Temperatursensoren, bestimmt wird und in einem zweiten Schritt wenigstens der zweite der Gasparameter in Abhängigkeit des ersten der Gasparameter, sowie insbesondere des Temperaturmesswerts an dem Temperatursensor oder an wenigstens einem weiteren der Temperatursensoren, bestimmt wird.

Bei einem solchen iterativen Verfahren ist es möglich, dass nur am Anfang des Verfahrens Temperaturmesswerte an den Temperatursensoren bestimmt werden. Es ist jedoch auch möglich, in jedem der Iterationsschritte oder zumindest in den Iterationsschritten, in denen ein erster der Parameter angepasst wird, die aktuellen Temperaturmesswerte der Temperatursensoren zu berücksichtigen.

Wie erläutert, ist häufig der Zusammenhang zwischen einem zu ermittelnden Gasparameter und dem dabei genutzten Temperaturwert bzw. der dabei genutzten Verknüpfung von Temperaturwerten nicht linear und von der Art des Gases oder ähnlichem abhängig. Daher ist es möglich, dass zur Bestimmung des Gasparameters oder wenigstens eines der Gasparameters eine, insbesondere mehrdimensionale, Wertetabelle genutzt wird, deren Werte in Abhängigkeit des jeweils einer Dimension der Wertetabelle zugeordneten Temperaturmesswerts wenigstens eines der Temperatursensoren, insbesondere interpoliert, ausgelesen werden. Solche Wertetabellen können bei einer Kalibrierung des Durchflussmessgeräts vor einem Einsatz zur Bestimmung von Gasparametern ermittelt werden.

Im einfachsten Fall können eine Vielzahl von Gasen bzw. Gasmischungen mit bekannten Parametern mit einer Vielzahl von Durchflussgeschwindigkeiten durch das Durchflussmessgerät geleitet werden und dabei können jeweils die Temperaturwerte an den Temperatursensoren ermittelt werden. Für eine Vielzahl von Gasen wird dabei ein Zusammenhang ermittelt werden, bei dem sich für jede Kombination von Temperaturmesswerten ein bestimmter Wert für einen Gasparameter ermitteln lässt. Ein solcher Wert kann auch eine Gasart, der Anteil eines Gases an einer Gasmischung oder eine Gasmischung selbst sein. Es kann sich bei den Einträgen dieser Tabellen also nicht ausschließlich um Zahlenwerte handeln. Es können in den Wertetabellen auch direkt alphanumerische Informationen bzw. Werte, die alphanumerischen Informationen zugeordnet sind, gespeichert sein.

Es ist jedoch auch möglich, dass die Temperaturmesswerte der Temperatursensoren den Gasparameter oder wenigstens einen der Gasparameter nicht eindeutig bestimmen, wodurch bei gleichen Temperaturmesswerten aller Temperatursensoren mehrere verschiedene Werte für den Gasparameter bestimmt werden, wobei die Auswahl des bestimmten Wertes von vorangehenden Temperaturmesswerten wenigstens eines der Temperatursensoren und/oder vorangehenden Werten des Gleichen oder eines weiteren der Gasparameter und/oder bekannten Betriebsbedingungen des Durchflussmessgeräts, insbesondere Informationen über zu erwartende Gaszusammensetzungen, abhängig ist.

Insbesondere kann das Durchflussmessgerät für eine Vielzahl von Gasen und/oder Gasmischungen kalibriert sein, das heißt, dass Gasparameter für große Gruppen von Gasen bzw. Gasgemischen bestimmt werden sollen. Dabei ist es möglich, dass für eine Kombination von ermittelten Temperaturen an den Temperatursensoren einem oder mehreren der Gasparameter kein eindeutiger Wert zugeordnet werden kann. So ist es beispielsweise möglich, dass abhängig von der Gasmischung, die durch den Durchflussmesser strömt, eine gewisse Temperaturverteilung einem großen Durchfluss bei einer großen Temperaturleitfähigkeit entspricht, jedoch für ein anderes Gas bei einem geringeren Gasfluss ähnliche Werte gemessen werden, beispielsweise, weil die Temperaturleitfähigkeit geringer ist und sich weitere nicht gemessene Gasparameter ebenfalls unterscheiden.

In diesen Fällen ist es insbesondere möglich, eine Konsistenzprüfung der Gasparameter durchzuführen. So kann beispielsweise bekannt sein, dass sich gewisse Gase nicht in der Gasmischung befinden, wodurch einige der Werte ausgeschlossen werden können. Die Recheneinrichtung selbst kann ohne zusätzliche externer Informationen bereits dadurch einen Konsistenzcheck durchführen, dass bei der Ermittlung der Gasparameter vorangehend ermittelte Gasparameter oder Werte an Temperatursensoren berücksichtigt werden. So ist es beispielsweise weit wahrscheinlicher, dass sich eine Gaszusammensetzung nur geringfügig ändert, als dass plötzlich eine vollständig andere Gaszusammensetzung durch den Durchflussmesser fließt.

Im Rahmen eines solchen Konsistenzchecks ist es möglich, gewisse der möglichen Gasparameter vollständig zu verwerfen, insbesondere wenn ermittelt wird, dass die Wahrscheinlichkeit unterhalb eines vorgegebenen Grenzwertes ist. Es ist aber auch möglich, den Wert des Gasparameters als eine gewichtete Summe der verschiedenen möglichen Werte für den Gasparameter zu bilden, wobei die Gewichtungsfaktoren abhängig von der Wahrscheinlichkeit der Korrektheit des Wertes für den Gasparameter sind.

Wie erwähnt, kann es häufig nachteilig sein große Wertetabellen zur Ermittlung von Gasparametern zu nutzen, da in Durchflussmessgeräten häufig Recheneinrichtungen mit sehr begrenzten Ressourcen eingesetzt werden. Daher kann es vorteilhaft sein, dass zur Berechnung des Gasparameters die Abhängigkeit des Gasparameters von den Temperaturwerten an den Temperatursensoren durch lineare oder polynominale Näherungen genähert wird. So kann beispielsweise ein unkorrigierter Durchfluss direkt aus der Differenz zwischen dem ersten und dem dritten Temperatursensor berechnet werden, wobei diese Differenz skaliert werden kann und zu der skalierten Differenz ein Offset addiert werden kann. Die Skalierung und der Offset können insbesondere abhängig von weiteren ermittelten Gasparametern sein. Ist von einem Gasparameter bekannt, dass er nicht ausreichend genau über den gesamten Wertebereich durch eine solche Näherung ausgedrückt werden kann, so ist es auch möglich, polynominale Näherungen zu nutzen, in denen beispielsweise Potenzen der Differenzen oder Summen von Temperaturmesswerten berücksichtigt werden. Es werden so höhere Potenzen von Temperaturmesswerten oder die Produkte von verschiedenen Temperaturmesswerten bei der Berechnung des Gasparameters genutzt.

Der Gasparameter oder einer der Gasparameter kann auch ein Mischverhältnis wenigstens zweier Gase, eine Konzentration eines Gastyps oder der Gastyp eines Gases sein. Dies ist insbesondere dann möglich, wenn bekannt ist, dass nur eine kleine Gruppe von Gasen durch die Messstrecke strömen kann. Die Ermittlung der genannten Parameter ist insbesondere vorteilhaft, da in diesem Fall beispielsweise je nach Art des Gases unterschiedliche Berechnungsmethoden für die weiteren Gasparameter genutzt werden können oder ähnliches. Zudem können durch Bestimmung des Gastyps bzw. des Mischungsverhältnisses auch weitere Parameter des Gases ermittelt werden, die im Durchflussmesser selbst nicht direkt ermittelt werden können. So ist es beispielsweise möglich, einen Brennwert eines Gases zu bestimmen, wodurch dann beispielsweise eine Abrechnung der Gasmengen nach tatsächlichem Brennwert statt einer reinen Volumenabrechnung möglich ist.

Um weitere Informationen über das duch das Durchflussmessgerät strömende Gas zu gewinnen und Gasparameter genauer zu bestimmen oder weitere Gasparameter zu bestimmen, können zusätzlich die Bedingungen, unter denen die Temperaturmesswerte bestimmt werden, geändert werden. Dabei kann insbesondere die Heizleistung des Heizelements zwischen einem ersten Wert und wenigstens einem zweiten Wert variiert werden, um wenigstens einen weiteren, unabhängigen Gasparameter zu bestimmen.

Insbesondere kann ein Temperaturmesswert wenigstens eines Temperatursensors oder ein daraus abgeleiteter Wert zu einem Zeitpunkt bestimmt werden, zu dem das Heizelement ausgeschaltet ist, wobei wenigstens ein Gasparameter in Abhängigkeit dieses Temperaturmesswertes bestimmt wird.

Im erfindungsgemäßen Verfahren werden durch die Auswertung von Temperaturmesswerten wenigstens dreier Temperatursensoren Zusatzinformationen über das Gas bereitgestellt. Dabei kann besonders vorteilhaft, insbesondere bei bekannter qualitativer Zusammensetzung des Gases, als der oder wenigstens einer der Gasparameter ein Brennwert des Gases bestimmt werden. Die Kombination einer Brennwertbestimmung mit einer Bestimmung der Durchflussmenge ermöglicht es insbesondere, eine Abrechnung einer gelieferten Gasmenge nicht ausschließlich nach dem Volumen des bereitgestellten Gases sondern nach dem Gesamtbrennwert des bereitgestellten Gases durchzuführen.

Des Weiteren betrifft die Erfindung ein Durchflussmessgerät, umfassend eine Recheneinrichtung und eine Messstrecke mit einem Heizelement und wenigstens drei Temperatursensoren über die das Gas geführt wird, wobei mindestens ein erster Temperatursensor stromaufwärts von dem Heizelement, mindestens ein zweiter Temperatursensor im Bereich des Heizsensors und mindestens ein dritter Temperatursensor stromabwärts von dem Heizelement angeordnet sind, wobei der zweite Temperatursensor insbesondere durch das Heizelement gebildet ist, und wobei die Recheneinrichtung zur Durchführung des vorangehend beschriebenen Verfahrens ausgebildet ist.

Dabei kann das Durchflussmessgerät insbesondere mehrere zweite Temperatursensoren umfassen, die stromaufwärts und stromabwärts direkt benachbart zum Heizelement angeordnet sind und einzeln oder gemeinsam einen Temperaturmesswert bereitstellen. Zusätzlich oder alternativ kann das Durchflussmessgerät auch mehrere erste und/oder mehrere dritte Temperatursensoren umfassen, die jeweils einzeln oder gemeinsam Temperaturmesswerte bereitstellen. Durch die Nutzung mehrer Temperatursensoren kann die Messqualität verbessert werden. Insbesondere ist es durch eine symmetrische Anordnung von zwei zweiten Temperatursensoren bezüglich des Heizelementes besonders gut möglich, Temperaturmessungen für den Bereich des Heizelements durchzuführen, ohne den Temperatursensor direkt am Heizelement selbst anordnen zu müssen.

Im erfindungsgemäßen Durchflussmesser können die Temperaturwerte der Temperatursensoren insbesondere direkt erfasst und analog/digital gewandelt werden, wobei die anschließende Weiterbearbeitung und Verknüpfung der Temperaturmesswerte zur Bestimmung der Gasparameter vollständig digital durchgeführt werden kann. Selbstverständlich ist es jedoch auch möglich, die Verknüpfung der Temperaturmesswerte zumindest teilweise durch separate analoge oder digitale Schaltungen durchzuführen. So ist es insbesondere möglich, dass das Durchflussmessgerät eine elektronische Schaltungseinheit umfasst, die den ersten oder wenigstens einen der ersten mit den zweiten oder wenigstens einem der zweiten und/oder dem dritten oder wenigstens einem der dritten der Temperatursensoren oder den zweiten oder wenigstens einen der zweiten mit dem dritten oder wenigstens einem der dritten Temperatursensoren derart verschaltet, dass wenigstens ein Ausgangssignal der Schaltungseinheit von den linear, insbesondere additiv oder subtraktiv und/oder multiplikativ und/oder dividierend verknüpften Temperaturmesswerten der durch die Schaltungseinheit verschalteten Temperatursensoren abhängt, wobei die Recheneinrichtung zur Bestimmung des oder wenigstens eines der Gasparameter in Abhängigkeit des Ausgangssignals der Schaltungseinheit ausgebildet ist.

Der Schutzumfang der Erfindung wird durch die Ansprüche definiert.

Weitere Vorteile und Einzelheiten der Erfindung ergeben sich aus den folgenden Ausführungsbeispielen sowie den zugehörigen Zeichnungen. Dabei zeigen:
- Fig. 1: schematisch ein Ausführungsbeispiel eines erfindungsgemäßen Durchflussgeräts,
- Fig. 2: schematisch den Ablauf eines Ausführungsbeispiels eines erfindungsgemäßen Verfahrens,
- Fig. 3: schematisch den Ablauf eines weiteren Ausführungsbeispiels eines erfindungsgemäßen Verfahrens,
- Fig. 4: schematisch den Ablauf eines dritten Ausführungsbeispiels eines erfindungsgemäßen Verfahrens,
- Fig. 5: schematisch den Ablauf eines vierten Ausführungsbeispiels eines erfindungsgemäßen Verfahrens
- Fig. 6: schematisch den Ablauf eines fünften Ausführungsbeispiels eines erfindungsgemäßen Verfahrens.

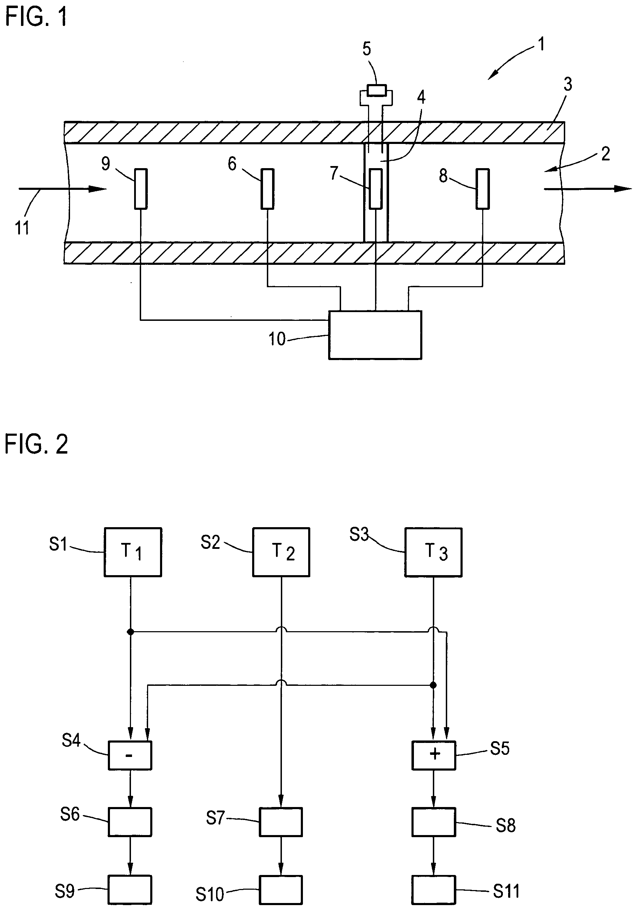

Fig. 1 zeigt ein Ausführungsbeispiel eines Durchflussgerätes zur Bestimmung wenigstens eines Gasparameters. Das Durchflussmessgerät 1 umfasst eine Messstrecke 2, die sich innerhalb einer Röhre 3 befindet. Die Röhre 3 ist hier beispielsweise als rechteckige Röhre mit einer geringen Höhe ausgebildet, um eine laminare Strömung zu erzeugen. Innerhalb der Messstrecke 2 ist zentral ein Heizelement 4 angeordnet, das über eine Steuerung 5 gesteuert werden kann. Die Steuerung 5 kann als separate Steuerung ausgebildet sein, sie kann aber auch über die Recheneinrichtung 10 steuerbar sein. Die Ansteuerung des Heizelements 4 durch die Steuerung 5 erfolgt typischerweise so, dass die Heizleistung des Heizelements 4 konstant ist. Um weitere Informationen über das Gas, das die Messstrecke 2 durchströmt, zu gewinnen, kann jedoch die Heizleistung des Heizelements 4 durch die Steuerung 5 auch zwischen zwei oder mehr Werten variiert werden. Hat das Heizelement 4 über einen weiten Temperaturbereich eine nahezu konstanten Widerstand, so kann es ausreichend sein, es durch die Steuerung 5 mit einer konstanten Spannung oder einem konstanten Strom zu beschicken, um eine konstante Heizleistung zu erreichen. Es ist jedoch auch möglich, dass die Steuerung 5 die Heizleistung auf einen konstanten Wert regelt.

Neben dem Heizelement 4 sind in der Messstrecke 2 drei Temperatursensoren 6, 7 und 8 angeordnet. Der Temperatursensor 7 ist im Bereich des Heizelements 4 angeordnet. Die beiden Temperatursensoren 6, 8 sind stromaufwärts, bzw. stromabwärts vom Heizelement angeordnet. Dabei zeigen die Pfeile 11 die Strömungsrichtung des Gases an. Die Messwerte der Temperatursensoren 6, 7 und 8 werden durch die Recheneinrichtung 10 aufgenommen. Dies kann beispielsweise über eine analog/digital-Wandlung erfolgen. Des Weiteren ist im Gasstrom, jedoch deutlich weiter als die Temperatursensoren 6, 8 von dem Heizelement 4 beabstandet, ein weiterer Temperatursensor 9 angeordnet, der die Umgebungstemperatur misst. Die Umgebungstemperatur entspricht ungefähr der Temperatur des einströmenden Gases und kann daher zur Präzisierung der ermittelten Gasparameter herangezogen werden.

Die Recheneinrichtung 10 nimmt die Temperaturen an den Temperatursensoren 6, 7, 8 und 9 auf und ermittelt aus den Temperaturwerten an den Temperatursensoren 6, 7, 8, 9 einen oder mehrere Gasparameter, wobei sie zur Ermittlung der Gasparameter zumindest die Temperaturwerte an den Temperatursensoren 6, 7 und 8 nutzt. Die Auswertung der Temperaturwerte an den Temperatursensoren 6, 7 und 8 wird im Folgenden mit Bezug auf Fig. 2 bis Fig. 6 beschrieben.

Fig. 2 zeigt beispielhaft eine mögliche Art der Ermittlung von Gasparametern aus den Temperaturwerten der in Fig. 1 gezeigten Temperatursensoren 6, 7 und 8. In den gleichzeitig oder in kurzem zeitlichen Abstand durchgeführten Schritten S1, S2 und S3 wird der Temperaturwert T1 am Temperatursensor 6, der Temperaturwert T2 an dem Temperatursensor 7 sowie der Temperaturwert T3 am Temperatursensor 8 aufgenommen. Zur Weiterverwertung dieser Werte wird ausgenutzt, dass verschiedene Durchflussmengen und verschiedene weitere Gasparameter zu einer unterschiedlichen Temperaturverteilung innerhalb der Messstrecke 2 führen. Die Temperatur wird typischerweise als ein glockenförmige Verteilung um das Heizelement 4 wieder auf die Umgebungstemperatur abfallen. Aus der Höhe, der Breite und der Asymmetrie der Temperaturverteilung können jedoch wesentliche Gasparameter bestimmt werden. So kann beispielsweise aus der Asymmetrie der Temperaturverteilung die Durchflussmenge ermittelt werden. Strömt kaum Gas durch die Messstrecke 2, so sollte die Temperatur in beide Richtungen vom Heizelement 4 ausgehend gleichmäßig abfallen, wodurch die Temperatur an den gleich weit vom Heizelement beabstandeten Temperatursensoren 6 und 8 ungefähr gleich sein sollte. Fließt jedoch Gas durch die Messstrecke 2, so führt dies dazu, dass warme Luft vom Heizelement 4 in Richtung des Temperatursensors 8 gedrückt wird. Dies führt dazu, dass der Temperaturwert T3 an dem Temperatursensor 8 erhöht wird. Steigt der Durchfluss weiter an, so sinkt der Temperaturwert T3 wieder. Zugleich kann weniger warme Luft vom Heizelement 4 zum Temperatursensor 6 diffundieren, weshalb die Temperatur T1 am Temperatursensor 8 sinkt.

Zur Berechnung des Gasflusses kann daher in Schritt S4 der Temperaturwert T1 vom Temperaturwert T3 abgezogen werden. Der Unterschied der Temperaturwerte T1 und T3 an den Temperatursensoren 6 und 8 ist ein gutes Maß für die Asymmetrie der Temperaturverteilung des Gases. Damit bildet diese Differenz auch ein gutes Maß für die Durchflussmenge.

Um Nichtlinearitäten und Offsets dieser Abhängigkeit zu berücksichtigen, kann der in Schritt S4 ermittelte Differenzwert in Schritt S6 als ein Index zum Auslesen einer Wertetabelle genutzt werden. Alternativ ist es auch möglich, in Schritt S6 den in Schritt S4 ermittelten Differenzwert mit einem Offset zu versehen und zu skalieren oder weitere Faktoren höherer Ordnung, beispielsweise des Quadrat der Differenz oder ähnliches hinzuzuaddieren.

Die Weiterverarbeitung des im Schritt S4 ermittelten Differenzwertes in Schritt S6 dient insbesondere auch dazu, zur Ausgabe oder Speicherung, in Schritt S9 Werte zu ermitteln, die in üblichen Einheiten, also beispielsweise cm³/s oder Ähnlichem angegeben sind. Die in Schritt S6 genutzten Skalierungs- und Offsetfaktoren bzw. die dort genutzte Wertetabelle kann im Rahmen der Kalibrierung des Durchflussmessgerätes festgelegt werden.

In dem gezeigten Ausführungsbeispiel wird zunächst davon ausgegangen, dass die Verarbeitung in Schritt S6 stets gleich erfolgt. Es sei jedoch bereits darauf hingewiesen, dass die Verarbeitung in Schritt S6 auch von den aktuell oder vorangehend gemessenen weiteren Gasparametern bzw. Temperaturwerten abhängig sein kann.

Ein weiterer Gasparameter, der einen Hinweis auf die gemessene Gassorte geben kann oder beispielsweise zur Korrektur der in Schritt S6 berechneten Durchflussmenge genutzt werden kann, ist die Wärmeleitfähigkeit des Gases bzw. der Gasmischung, die die Messstrecke 2 durchströmt. In dem gezeigten Verfahren wird das Heizelement 4 typischerweise mit einer konstanten Leistung beheizt. Damit ist die Temperatur des Heizelementes 4 bzw. die am Heizelement 4 mit dem Temperatursensor 7 in Schritt S2 gemessenen Temperatur T2 abhängig davon, wie schnell Wärme vom Temperatursensor 4 abtransportiert werden kann. Ist die Wärmeleitfähigkeit eines Gases groß, so kann ein starker Wärmetransport erfolgen und die Temperatur T2 ist kleiner als in dem Fall, in dem die Wärmeleitfähigkeit sehr klein ist und damit kaum Wärmetransport vom Heizelement 4 weg erfolgt. Damit kann die in Schritt S2 gemessene Temperatur T2 direkt als ein Maß für die Wärmeleitfähigkeit des durch die Messstrecke 2 strömenden Gases genutzt werden, insbesondere wenn ds Gas in Ruhe ist.

Der in Schritt S2 gemessene Wert kann in Schritt S7 weiterverarbeitet werden, um Offsets und Skalierungen zu berücksichtigen oder es kann in Schritt S7 eine Wertetabelle genutzt werden, um Nichtlinearitäten im Zusammenhang zwischen dem Temperaturwert T2 und der Wärmeleitfähigkeit zu berücksichtigen. Zur Ermittlung und Nutzung dieser Tabellen gilt das zu Schritt S6 Gesagte.

Anschließend kann der in Schritt S7 ermittelte Wert in Schritt S10 ausgegeben oder gespeichert werden. Auch hier ist es möglich, den ermittelten Wert in Abhängigkeit der weiteren ermittelten Gasparameter zu korrigieren, was im Weiteren noch genauer erläutert wird.

Neben der Höhe der Temperaturverteilung, die wie erläutert ein Maß für die Wärmeleitfähigkeit ist, und der Asymmetrie der Temperaturverteilung, die wie erläutert ein Maß für die Durchflussmenge ist, kann auch die Breite der Temperaturverteilung ermittelt werden. Die Breite der Temperaturverteilung kann als ein Maß dafür angesehen werden, wie schnell ein Temperaturtransport in dem Gas erfolgt, also ein Maß für die Temperaturleitfähigkeit. Eine größere Breite der Temperaturverteilung führt dazu, dass die Temperaturwerte an den Temperatursensoren 6 und 8, also die Temperaturwerte T1 und T3 steigen. Als Maß für die Breite kann beispielsweise die Summe der Temperaturwerte T1 und T3 betrachtet werden. Diese Summe wird in Schritt S5 aus den in Schritt S1 und Schritt S3 gemessenen Temperaturwerten T1 und T3 gebildet. Auch hier erfolgt in Schritt S8 eine Weiterverarbeitung, für die das zu Schritt S6 und Schritt S7 Gesagte gilt. Der ermittelte Wert für die Temperaturleitfähigkeit wird in Schritt S11 ausgegeben oder gespeichert.

Bereits mit dem gezeigten, relativ einfach zu implementierenden Verfahren können neben der Durchflussmenge zusätzliche Gasparameter, nämlich beispielsweise die Temperaturleitfähigkeit und die Wärmeleitfähigkeit ermittelt werden. Neben einem direkten Darstellen oder Speichern dieser Werte in den Schritten S9, S10 und S11 ist es selbstverständlich möglich, den Wert wenigstens eines dieser Gasparameter in Abhängigkeit der weiteren ermittelten Gasparameter zu korrigieren. Es ist auch möglich, dass die weiteren ermittelten Gasparameter bereits in den Verarbeitungsschritten S6, S7 und S8 berücksichtigt werden. So kann beispielsweise in Schritt S6 zwischen mehreren Wertetabellen zur Berechnung der Durchflussmenge gewählt bzw. interpoliert werden, wobei die Wahl bzw. der Interpolationspunkt zwischen den Tabellen von der ermittelten Wärme- und Temperaturleitfähigkeit abhängt. Des Weiteren können bei der Berechnung ebenfalls noch die Werte von weiteren Sensoren, also beispielsweise der Temperaturwert des weiteren Temperatursensors 9 genutzt werden. Diese komplexeren Verknüpfungen werden im Folgenden mit Bezug auf die weiteren Figuren erläutert.

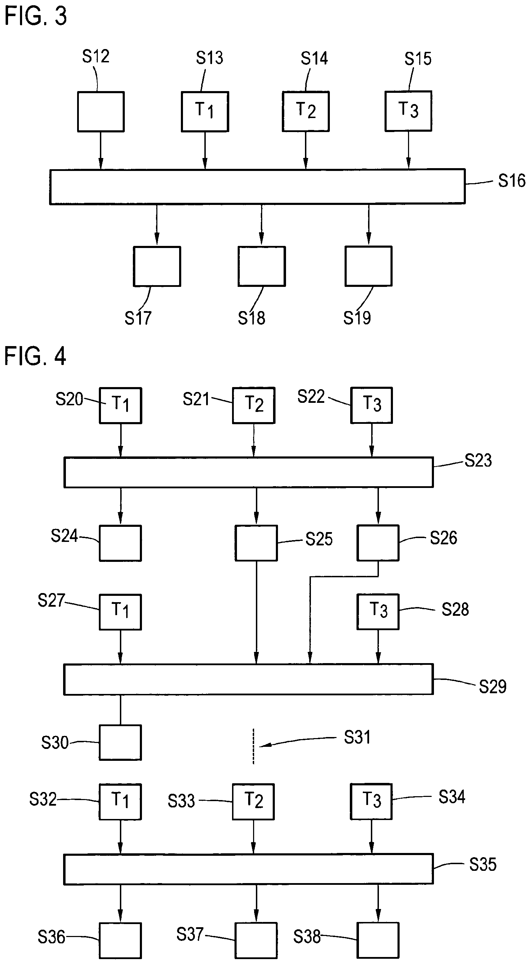

Fig. 3 zeigt beispielhaft ein Verfahren, in dem einer oder mehrere Parameter in Abhängigkeit von mehreren Messwerten ermittelt werden. Zunächst werden in Schritt S12, S13, S14 und S15 Messwerte ermittelt. In den Schritten S13, S14 und S15 werden die Temperaturen T1, T2 und T3 ermittelt, wie bereits zu Schritt S1 bis S3 erläutert. In Schritt S12 wird zudem der Temperaturwert am weiteren Temperatursensor 9 ermittelt. Selbstverständlich können in Schritt S12 oder einem weiteren Schritt auch andere ergänzende Parameter, beispielsweise die Temperaturen an den Temperatursensoren 6, 7 und 8 bei einer anderen Heizleistung am Heizelement, die Temperatur an einem weiteren Temperatursensor, der an einer vorgegebenen Position der Messstrecke angeordnet ist, bekannte Werte über Gaszusammensetzungen oder Ähnliches ermittelt werden. Damit werden in den Schritten S12 bis S15 mehrere, insbesondere vier Parameter bestimmt, die anschließend als Indizes zum Nachschlagen in einer mehrdimensionalen Wertetabelle genutzt werden. Werden beispielsweise die Temperaturen T1, T2, T3 sowie die Temperatur am weiteren Temperatursensor 9 genutzt, so ergibt sich eine vierdimensionale Wertetabelle für jeden zu ermittelnden Gasparameter. Beispielhaft ist hier die Ermittlung von drei Gasparametern in den Schritten S17, S18 und S19 gezeigt. Das Prinzip soll nur für die Ermittlung eines einzelnen Gasparameters erläutert werden. Weitere Gasparameter werden äquivalent ermittelt.

Soll beispielsweise ausschließlich eine Durchflussmenge ermittelt werden, so kann hierfür eine Wertetabelle genutzt werden, die beispielsweise während eines Kalibrierprozesses des Durchflussmessgeräts 1 ermittelt wurde. Für einen solchen Kalibrierprozess kann das Durchflussmessgerät 1 mit einer Vielzahl von unterschiedlichen Gassorten bei unterschiedlichen Strömungsgeschwindigkeiten und Umgebungsbedingungen durchströmt werden, wobei jeweils die Temperaturwerte der Temperatursensoren 6, 7 und 8 sowie des weiteren Temperatursensors aufgenommen werden. Für eine Vielzahl von Gasgruppen ist es in diesem Fall möglich, eine eindeutige Bestimmung der Durchflussmenge aus den Temperaturwerten T1, T2 und T3 sowie der Temperatur am weiteren Temperatursensor 9 zu bestimmen. In Einzelfällen kann es jedoch möglich sein, dass für verschiedene Durchflussmengen bei der Kalibrierung die gleiche Temperaturkombination an den Temperatursensoren 6, 7, 8 und 9 festgestellt wurden, beispielsweise wenn verschiedene Gaszusammensetzungen genutzt wurden. Zur Auflösung solcher Uneindeutigkeiten ist es bei dem Messprozess möglich, dass neben den aktuell gemessenen Temperaturwerten T1, T2, T3 noch vorangehende Temperaturmesswerte, zusätzliche bekannte Parameter wie beispielsweise die Art des den Durchflussmesser durchströmenden Gases, vorangehend ermittelte Gasparameter oder Ähnliches, genutzt werden.

Gegenüber üblichen Durchflussmessgeräten ist durch die Berücksichtigung der Temperaturen T1, T2 und T3 sowie des weiteren Temperatursensors 9 eine deutlich genauere Bestimmung der Durchflussmenge möglich. Gleiches gilt selbstverständlich auch für andere Parameter. Sollen mehrere Parameter bestimmt werden, so wird schlicht für jeden der Parameter eine getrennte mehrdimensionale Messwertetabelle genutzt.

Alternativ oder ergänzend zur Bestimmung eines oder mehrerer Parameter durch Nutzung einer Wertetabelle ist es bei Bestimmung mehrerer Parameter aus den Temperaturwerten T1, T2 und T3 sowie dem Temperaturwert des weiteren Temperatursensors 9 auch möglich, dass einer der Gasparameter in Abhängigkeit eines zugleich oder vorangehend bestimmten Gasparameters bestimmt wird. Damit können gegenseitige Abhängigkeiten zwischen den Gasparametern korrigiert werden. So kann beispielsweise ein korrigierter, eine Wärmeleitfähigkeit beschreibender Gasparameter berechnet werden, indem zunächst ein unkorrigierter, eine Wärmeleitfähigkeit beschreibender Gasparameter und ein eine Durchflussmenge beschreibender Gasparameter bestimmt werden und der korrigierte Gasparameter in Abhängigkeit dieser beiden Parameter berechnet wird. Alternativ ist es auch möglich, einen Gasparameter in Abhängigkeit mehrerer weiterer bestimmter Gasparameter zu bestimmen. So kann als Gasparameter beispielsweise ein eine Temperaturleitfähigkeit beschreibender Gasparameter bestimmt werden, wobei dieser in Abhängigkeit eines eine Wärmeleitfähigkeit beschreibenden Gasparameters und eines eine Durchflussmenge beschreibenden Gasparameters bestimmt wird.

Fig. 4 zeigt ein drittes Ausführungsbeispiel zur Ermittlung eines Durchflusses, in dem verschiedene Gasparameter mit verschiedener Häufigkeit gemessen werden. Dies ist vorteilhaft, da dadurch der Energieverbrauch eines Durchflussmessgerätes gesenkt werden kann. Insbesondere Gaszähler werden häufig durch Batterien oder ähnliches mit Energie versorgt und sollen lange Zeit ohne einen Batteriewechsel funktionieren. Daher ist eine Optimierung des Stromverbrauchs von Gaszählern und anderen Durchflussmessgeräten wünschenswert. Die Schritte S20 bis S26 zeigen hier ein erstes Ermitteln dreier Gasparameter. Die Ermittlung der drei Gasparameter erfolgt im Wesentlichen wie in Fig. 3 gezeigt, allerdings ist hier nur eine Nutzung der Temperaturwerte T1, T2 und T3 gezeigt. Selbstverständlich ist dieses Verfahren auch dann anwendbar, wenn die Temperaturwerte eines weiteren Temperatursensors 9 oder weitere Messwerte im Verfahren genutzt werden. Aus den aufgenommenen Temperaturwerten T1, T2 und T3 werden in Schritt S23 durch Nutzung dreier dreidimensionaler Wertetabellen drei Gasparameter ermittelt, die in Schritt S24, S25 und S26 ausgegeben, gespeichert oder Ähnliches werden. Selbstverständlich ist es auch möglich, in Schritt 23 statt mehreren mehrdimensionalen Wertetabellen einfachere Zusammenhänge zwischen den Temperaturmesswerten T1, T2 und T3 und den Gasparametern zu nutzen, wie beispielsweise in Zusammenhang mit Fig. 2 erläutert.

Ist nun davon auszugehen, dass sich einer der Gasparameter, beispielsweise eine Durchflussmenge, erheblich schneller ändert als die weiteren Gasparameter, beispielsweise die Temperaturleitfähigkeit und/oder die Wärmeleitfähigkeit und damit die Zusammensetzung des das Durchflussmessgerät 1 durchströmenden Gases, so kann es ausreichend sein, im Folgenden einen oder mehrere Parameter, also beispielsweise die Durchflussmenge in relativ kurzem Abstand wiederholt zu messen und die weiteren Gasparameter erst nach längerem Zeitabstand erneut zu ermitteln.

Das Ermitteln des schneller veränderlichen Gasparameters ist in den Schritten S27 bis S30 gezeigt. In den Schritten S27 und S28 werden die Temperaturwerte T1 und T3 an den Temperatursensoren 6 und 8 erneut ermittelt. Aus diesen beiden Gaswerten kann Schritt S29 beispielsweise eine Durchflussmenge bestimmt werden, die in Schritt S30 ausgegeben, gespeichert oder Ähnliches wird. Es ist möglich, dass die Ermittlung in Schritt S29 ausschließlich aus den Schritt S27 und Schritt S28 gemessenen Temperaturen erfolgt. Dies kann beispielsweise durch Bestimmen einer Differenz der Temperaturmesswerte T1 und T3 und ein entsprechendes Weiterverarbeiten, wie im Zusammenhang mit Fig. 2 erläutert, erfolgen. Es kann auch eine zweidimensionale Wertetabelle genutzt werden.

Es ist jedoch auch möglich, die in Schritt S25 und Schritt S26 bestimmten weiteren Gasparameter bei der Ermittlung der Durchflussmenge in Schritt S29 zu nutzen. Nachdem davon ausgegangen wird, dass diese beiden Gasparameter langsam veränderlich sind, können die jeweils ermittelten Parameter für mehrere Ermittlungen der Durchflussmenge genutzt werden.

Die Schritte S27 bis S30 können mehrmals wiederholt werden. Dieses wird als Schritt S31 angedeutet. Nach mehrmaliger Ermittlung des schneller veränderlichen Gasparameters sollen jedoch die langsam veränderlichen Gasparameter erneut ermittelt werden, was in Schritt S32 bis S38 erfolgt. Diese Bestimmung entspricht der Bestimmung in den Schritten S20 bis S26.

Insbesondere durch die Nutzung vorangehend ermittelter Werte für die langsam veränderlichen Gasparameter bei der mehrmaligen Ermittlung des schnell veränderlichen Gasparameters kann eine deutliche Verbesserung der Genauigkeit der Ermittlung des schnell veränderlichen Gasparameters erreicht werden, ohne dass die langsam veränderlichen Gasparameter mit der gleichen Häufigkeit gemessen werden müssen.

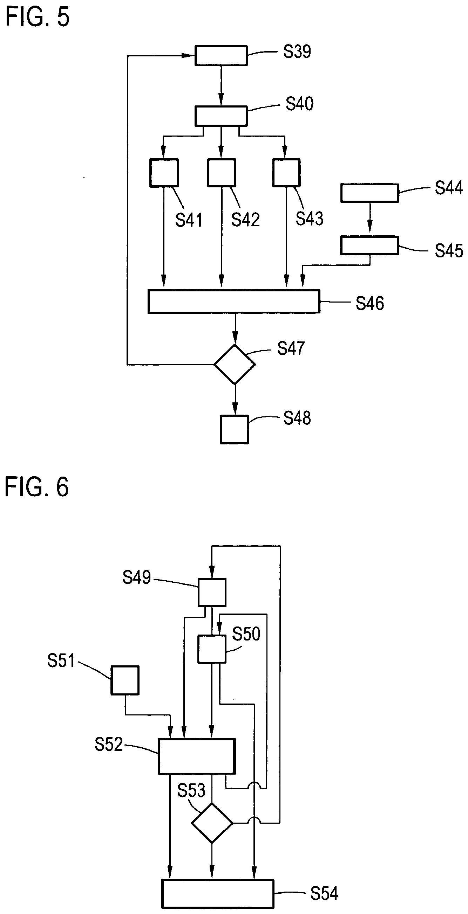

Wie bereits erwähnt, kann es möglich sein, dass bei der Kalibrierung des Durchflussmessgeräts festgestellt wird, dass mehrere Werte eines oder mehrerer Gasparameter zu den gleichen Temperaturwerten T1, T2 und T3 sowie den gleichen Werten an weiteren Sensoren führen. In solchen Fällen soll ermittelt werden, welcher der Werte der zutreffende Wert ist. Ein Beispiel für eine solche Ermittlung ist in Fig. 5 gezeigt. Hier werden in Schritt S39 zunächst alle verfügbaren Messwerte, also insbesondere die Temperaturwerte T1, T2 und T3 aufgenommen. In Schritt S40 erfolgt eine Auswertung dieser Messwerte, um wenigstens einen Gasparameter zu ermitteln. Verschiedene Möglichkeiten für diese Auswertung sind bereits mit Bezug auf Fig. 2 bis 4 erläutert und werden zudem noch mit Bezug auf Fig. 6 erläutert werden. Zur größeren Übersichtlichkeit ist in Fig. 5 nur die Ermittlung eines Gasparameters dargestellt.

Nun ist es möglich, dass in Schritt S40 der Gasparameter nicht eindeutig ermittelt wird. Hier wird beispielhaft die Ermittlung dreier möglicher Gasparameter in den Schritten S41, S42 und S43 gezeigt. Um diese Uneindeutigkeit aufzulösen, kann es vorteilhaft sein, zusätzliche Informationen zu gewinnen. Daher ist es möglich, dass in Schritt S44 das Heizelement 4 mit einer anderen Leistung geheizt wird und im Schritt S45 weitere Werte für die in Schritt S40 gemessenen Messparameter aufgenommen werden. Das weitere Verfahren ist aber auch ohne diese zusätzlichen Schritte möglich.

In Schritt S46 wird versucht zu ermitteln, welcher der in Schritt S41, S42 und S43 ermittelten Gasparameter der korrekte Gasparameter ist. Hierbei ist es möglich, die zusätzlichen nach dem Heizen gewonnenen Messwerte zu nutzen, es ist aber auch möglich, dass die ermittelten Werte für weitere Gasparameter genutzt werden oder dass beispielsweise Informationen über mögliche Gaszusammensetzungen vorliegen, die in diesem Schritt genutzt werden können.

Zudem ist es möglich, dass zuvor ermittelte Werte für den Gasparameter oder zuvor gemessene Temperaturwerte gespeichert sind und in Schritt S46 zur Bestimmung des tatsächlichen Gasparameters genutzt werden können. In Schritt S47 wird überprüft, ob ein eindeutiger Parameter bestimmt werden konnte. Ist dies nicht der Fall, ist es möglich das Verfahren von Anfang an zu wiederholen. Dadurch können Parameter über längere Zeiträume aufgenommen werden, wodurch es möglich sein kann, zwischen verschiedenen Möglichkeiten für den Gasparameter zu unterscheiden. Ist es über längere Zeit hin nicht möglich, einen Wert zu ermitteln, so ist es möglich einen Fehler auszugeben, der darauf hinweist, dass zur Auswertung weitere Parameter notwendig sind, es kann aber auch ein Durchschnittswert der verschiedenen Möglichkeiten für den Gasparameter gebildet werden. Es ist auch möglich, dass durch Nutzung vorangehender Messwerte und zusätzlicher Messdaten und Informationen Wahrscheinlichkeiten für die verschiedenen Werte des Gasparameters ermittelt werden und der in Schritt S48 ermittelte, ausgegebene bzw. gespeicherte Gasparameter als eine mit den Wahrscheinlichkeiten gewichtete Summe dieser möglichen Gasparameter berechnet wird.

In den vorangegangenen Ausführungsbeispielen wurde davon ausgegangen, dass Gasparameter aus einem einzelnen Temperaturwert oder einer Summe oder Differenz von mehreren Temperaturwerten ermittelt werden oder dass eine mehrdimensionale Tabelle zur Bestimmung des Gasparameters genutzt wird. Die Nutzung von mehrdimensionalen Tabellen erfordert große Mengen von Speicher in der Recheneinrichtung und die Ermittlung einer solchen mehrdimensionalen Kalibriertabelle ist aufwendig. Andererseits ist die direkte Bestimmung der Gasparameter aus einzelnen Temperaturwerten oder Summen oder Differenzen von Temperaturparametern häufig nicht ausreichend, um eine gewünschte Genauigkeit zu erreichen. Daher kann es in einigen Fällen vorteilhaft sein, die Gasparameter zwar zunächst einzeln zu bestimmen, anschließend jedoch in Abhängigkeit der weiteren Gasparameter zu korrigieren.

Da nun typischerweise die ermittelten Werte eines ersten Gasparameters von den tatsächlichen Werten eines zweiten Gasparameters abhängen und die ermittelten Werte eines zweiten Gasparameters von den tatsächlichen Werten eines ersten Gasparameters ist eine wechselseitige Abhängigkeit gegeben, die nicht leicht aufgelöst werden kann. Daher kann es vorteilhaft sein, die Werte für die Gasparameter iterativ zu berechnen.

Ein Beispiel für eine solche iterative Berechnung ist in Fig. 6 gezeigt. Hier werden in Schritt S49 zunächst einer oder mehrere Messwerte, insbesondere Temperaturmesswerte T1, T2 und/oder T3 aufgenommen, aus denen in Schritt S50 ein erster Gasparameter bestimmt wird. Beispielsweise kann eine solche Bestimmung eines ersten Gasparameters hier durch eine einfache Summe oder Differenz von Temperaturwerten, wie mit Bezug auf Fig. 2 erläutert, erfolgen. Nach der Ermittlung des ersten Gasparameters können in Schritt S51 weitere Messwerte aufgenommen werden. Dies können aktuelle Werte der bereits in Schritt S49 ausgelesenen Temperatursensoren sein, es können jedoch auch andere Temperatursensoren ausgelesen werden oder weitere Werte genutzt werden. In Schritt S52 wird nun ein zweiter Gasparameter bestimmt. Zur Bestimmung des zweiten Gasparameters können zum einen die in Schritt S52 neu aufgenommenen Werte, zum anderen die bereits in Schritt S49 aufgenommenen Werte genutzt werden. Zusätzlich wird bei der Bestimmung des zweiten Gasparameters in Schritt S52 der Wert des ersten Gasparameters, der in Schritt S50 bestimmt wurde, berücksichtigt. In Schritt S52 wird also ein zweiter Gasparameter aus Messwerten und einem ersten Gasparameter bestimmt.

In Schritt S53 kann eine Abbruchbestimmung geprüft werden, die beispielsweise ein Konvergenzkriterium sein kann, beispielsweise dass zwei aufeinanderfolgend ermittelte Werte für einen Gasparameter einen gewissen maximalen Abstand nicht übersteigen. Ist dieses Konvergenzkriterium nicht erfüllt, so wird das Verfahren ab Schritt S49 wiederholt, in Schritt S50 wird jedoch zusätzlich zur Berechnung des ersten Gasparameters der in Schritt S52 bestimmte zweite Gasparameter der vorangehenden Iteration genutzt. Es ist zudem auch möglich die Temperaturmesswerte vorangehender Iterationen zu nutzen. Sobald das Konvergenzkriterium in Schritt S53 erfüllt wird, werden die zuletzt ermittelten ersten und zweiten Gasparameter in Schritt S54 ausgegeben.

Offensichtlich ist dieses Verfahren auch zur Ermittlung weiterer Gasparameter erweiterbar, so können beispielsweise in Schritt S50 und/oder in Schritt S52 mehrere Gasparameter bestimmt werden.

Bei dem mit Bezug auf Fig. 6 beschriebenen Ermittlungsverfahren handelt es sich um ein selbstkonsistentes Verfahren, das heißt um ein Verfahren, bei dem mehrere sich gegenseitig beeinflussende Parameter iterativ so berechnet werden, dass die Werte der Parameter nach einigen Durchgängen konvergieren.

Für einen Fachmann ist es ein Leichtes, die verschiedenen angegebenen Ausführungsbeispiele zu kombinieren. So ist beispielsweise eine Kombination von iterativen Verfahren mit der Nutzung von mehrdimensionalen Wertetabellen zur Ermittlung von Werten möglich, eine Iteration kann auch im Rahmen der Konsistenzbestimmung genutzt werden und die Werte zusätzlicher Sensoren lassen sich leicht in das Verfahren integrieren.

## Patentansprüche

1. Verfahren zum Bestimmen wenigstens eines Gasparameters, insbesondere einer Durchflussmenge, eines strömenden Gases, mittels eines Durchflussmessgeräts, umfassend eine Messstrecke mit einem Heizelement und wenigstens drei Temperatursensoren, über die das Gas geführt wird, wobei mindestens ein erster Temperatursensor stromaufwärts von dem Heizelement, mindestens ein zweiter Temperatursensor im Bereich des Heizelements und mindestens ein dritter Temperatursensor stromabwärts von dem Heizelement angeordnet sind, wobei insbesondere das Heizelement selbst als zweiter Temperatursensor genutzt werden kann, wobei eine Recheneinrichtung den wenigstens einen Gasparameter in Abhängigkeit der Temperaturmesswerte am ersten und am zweiten und am dritten Temperatursensor bestimmt, und/oder wobei die Recheneinrichtung wenigstens zwei separate Gasparameter in Abhängigkeit der Temperaturmesswerte einzelner unterschiedlicher der Temperatursensoren und/oder der Kombinationen von Temperaturmesswerten unterschiedlicher der Temperatursensoren bestimmt, wobei zur Bestimmung der Gasparameter die Temperaturmesswerte am ersten und am zweiten und am dritten Temperatursensor verwendet werden,
**dadurch gekennzeichnet,**
**dass** wenigstens zwei der Gasparameter in einem iterativen Verfahren ermittelt werden, wobei abwechselnd in einem ersten Schritt wenigstens ein erster der Gasparameter in Abhängigkeit wenigstens eines zweiten der Gasparameter bestimmt wird und in einem zweiten Schritt wenigstens der zweite der Gasparameter in Abhängigkeit des ersten der Gasparameter bestimmt wird, wobei in dem ersten Schritt der erste der Gasparameter in Abhängigkeit des Temperaturmesswertes an wenigstens einem der Temperatursensoren bestimmt wird und/oder wobei in dem zweiten Schritt der zweite der Gasparameter in Abhängigkeit des Temperaturmesswerts an dem Temperatursensor oder an wenigstens einem weiteren der Temperatursensoren bestimmt wird.

2. Verfahren nach Anspruch 1,
**dadurch gekennzeichnet,**
**dass** in Abhängigkeit von den Temperaturmesswerten der wenigstens drei Temperatursensoren drei unabhängige Gasparameter bestimmt werden, die durch die Recheneinrichtung in Abhängigkeit dreier linear unabhängiger Linearkombinationen der Temperaturmesswerte des ersten, des zweiten und des dritten Temperatursensors bestimmt werden.

3. Verfahren nach Anspruch 1 oder 2,
**dadurch gekennzeichnet,**
**dass** durch wenigstens einen vierten Temperatursensor die Umgebungstemperatur und/oder die Gastemperatur außerhalb des Messbereichs der wenigstens drei Temperatursensoren und beabstandet von dem Heizelement, insbesondere vor Eintritt des Gases in den Messbereich des ersten Temperatursensors und/oder nach Austritt aus dem Messbereich des dritten Temperatursensors, gemessen wird.

4. Verfahren nach einem der vorangehenden Ansprüche,
**dadurch gekennzeichnet,**
**dass** bei Bestimmung wenigstens zweier Gasparameter die Gasparameter wiederholt bestimmt werden, wobei für wenigstens einen der Gasparameter die Bestimmung mit einem anderen zeitlichen Abstand wiederholt wird als für wenigstens einen anderen der Gasparameter.

5. Verfahren nach einem der vorangehenden Ansprüche,
**dadurch gekennzeichnet,**
**dass** als einer der Gasparameter eine unkorrigierte Durchflussmenge aus den Temperaturmesswerten am ersten und am dritten Temperatursensor bestimmt wird, wobei aus der unkorrigierten Durchflussmenge und wenigstens einem weiteren der ermittelten Gasparameter eine korrigierte Durchflussmenge bestimmt wird.

6. Verfahren nach einem der vorangehenden Ansprüche,
**dadurch gekennzeichnet,**
**dass** wenigstens einer der ermittelten Gasparameter eine Wärmeleitfähigkeit des Gases oder eine Temperaturleitfähigkeit des Gases beschreibt.

7. Verfahren nach einem der vorangehenden Ansprüche,
**dadurch gekennzeichnet,**
**dass** als einer der Gasparameter ein korrigierter, eine Wärmeleitfähigkeit beschreibender Gasparameter in Abhängigkeit eines unkorrigierten, eine Wärmeleitfähigkeit beschreibenden Gasparameters und eines eine Durchflussmenge beschreibenden Gasparameters bestimmt wird, und/oder dass als einer der Gasparameter ein eine Temperaturleitfähigkeit beschreibender Gasparameter in Abhängigkeit eines eine Wärmeleitfähigkeit beschreibenden Gasparameters und eines eine Durchflussmenge beschreibenden Gasparameters, die jeweils in Abhängigkeit wenigstens eines momentanen oder vorab erfassten Temperaturmesswertes bestimmt werden, bestimmt wird.

8. Verfahren nach einem der vorangehenden Ansprüche,
**dadurch gekennzeichnet,**
**dass** zur Bestimmung des Gasparameters oder wenigstens eines der Gasparameter eine, insbesondere mehrdimensionale, Wertetabelle genutzt wird, deren Werte in Abhängigkeit des jeweils einer Dimension der Wertetabelle zugeordneten Temperaturmesswerts wenigstens eines der Temperatursensoren, insbesondere interpoliert, ausgelesen werden.

9. Verfahren nach einem der vorangehenden Ansprüche,
**dadurch gekennzeichnet,**
**dass** die Temperaturmesswerte der Temperatursensoren den Gasparameter oder wenigstens einen der Gasparameter nicht eindeutig bestimmen, wodurch bei gleichen Temperaturmesswerten aller Temperatursensoren mehrere verschiedene Werte für den Gasparameter bestimmt werden, wobei die Auswahl des bestimmten Wertes von vorangehenden Temperaturmesswerten wenigstens eines der Temperatursensoren und/oder vorangehenden Werten des gleichen oder eines weiteren der Gasparameter und/oder bekannten Betriebsbedingungen des Durchflussmessgeräts, insbesondere Informationen über zu erwartende Gaszusammensetzungen, abhängig ist.

10. Verfahren nach einem der vorangehenden Ansprüche,
**dadurch gekennzeichnet,**
**dass** zur Berechnung des Gasparameters die Abhängigkeit des Gasparameters von den Temperaturwerten an den Temperatursensoren durch lineare oder polynomiale Näherungen genähert wird.

11. Verfahren nach einem der vorangehenden Ansprüche,
**dadurch gekennzeichnet,**
**dass** der Gasparameter oder einer der Gasparameter ein Mischverhältnis wenigstens zweier Gase, eine Konzentration eines Gasestyps oder der Gastyp eines Gases ist, und/oder dass, insbesondere bei bekannter qualitativer Zusammensetzung des Gases, als der oder wenigstens einer der Gasparameter ein Brennwert des Gases bestimmt wird.

12. Verfahren nach einem der vorangehenden Ansprüche,
**dadurch gekennzeichnet,**
**dass** die Heizleistung des Heizelements zwischen einem ersten Wert und wenigstens einem zweiten Wert variiert wird, um wenigstens einen weiteren, unabhängigen Gasparameter zu bestimmen.

13. Verfahren nach einem der vorangehenden Ansprüche,
**dadurch gekennzeichnet,**
**dass** ein Temperaturmesswert wenigstens eines Temperatursensors oder ein daraus abgeleiteter Wert zu einem Zeitpunkt bestimmt wird, zu dem das Heizelement ausgeschaltet ist, wobei wenigstens ein Gasparameter in Abhängigkeit dieses Temperaturmesswertes bestimmt wird.

14. Durchflussmessgerät umfassend eine Recheneinrichtung (10) und eine Messstrecke (2) mit einem Heizelement (4) und wenigstens drei Temperatursensoren (6, 7, 8), über die das Gas geführt wird, wobei mindestens ein erster Temperatursensor (6) stromaufwärts von dem Heizelement (4), mindestens ein zweiter Temperatursensor (7) im Bereich des Heizelements (4) und mindestens ein dritter Temperatursensor (8) stromabwärts von dem Heizelement (4) angeordnet sind, und wobei der zweite Temperatursensor (7) insbesondere durch das Heizelement (4) gebildet ist,
**dadurch gekennzeichnet,**
**dass** die Recheneinrichtung (10) zur Durchführung des Verfahrens nach einem der Ansprüche 1 bis 13 ausgebildet ist.

15. Durchflussmessgerät nach Anspruch 14,
**dadurch gekennzeichnet,**
**dass** es mehrere zweite Temperatursensoren (7) umfasst, die stromaufwärts und stromabwärts direkt benachbart zum Heizelement (4) angeordnet sind und einzeln oder gemeinsam einen Temperaturmesswert bereitstellen.

16. Durchflussmessgerät nach Anspruch 14 oder 15,
**dadurch gekennzeichnet,**
**dass** es eine elektronische Schaltungseinheit umfasst, die den ersten oder wenigstens einen der ersten mit dem zweiten oder wenigstens einem der zweiten und/oder dem dritten oder wenigsten einem der dritten der Temperatursensoren (6, 7, 8) oder den zweiten oder wenigstens einen der zweiten mit dem dritten oder wenigstens einem der dritten der Temperatursensoren (7, 8) derart verschaltet, dass wenigstens ein Ausgangssignal der Schaltungseinheit von den linear, insbesondere additiv oder subtraktiv, und/oder multiplikativ und/oder dividierend verknüpften Temperaturmesswerten der durch die Schaltungseinheit verschalteten Temperatursensoren (6, 7, 8) abhängt, wobei die Recheneinrichtung (10) zur Bestimmung des oder wenigstens eines der Gasparameter in Abhängigkeit des Ausgangssignals der Schaltungseinheit ausgebildet ist.

## Claims

1. Method for determining at least one gas parameter, in particular a flow rate, of a flowing gas, by means of a flow meter, comprising a measuring section with a heating element and at least three temperature sensors over which the gas is guided, wherein at least one first temperature sensor is arranged upstream of the heating element, at least one second temperature sensor is arranged in the region of the heating element and at least one third temperature sensor is arranged downstream of the heating element, wherein in particular the heating element itself can be used as a second temperature sensor, wherein a computing device determines the at least one gas parameter as a function of the temperature measured values at the first and second and third temperature sensor, and/or wherein the computing device determines at least two separate gas parameters as a function of the temperature measured values of different individual temperature sensors and/or of the combinations of temperature measured values of different temperature sensors, wherein the temperature measured values at the first, second and third temperature sensor are used to determine the gas parameters,
**characterized**
**in that** at least two of the gas parameters are determined in an iterative method, wherein in a first step, alternately, at least one first parameter of the gas parameters is determined as a function of at least one second parameter of the gas parameters, and in a second step at least the second of the gas parameters is determined as a function of the first of the gas parameters, wherein in the first step the first of the gas parameters is determined as a function of the temperature measured values at at least one of the temperature sensors, and/or wherein in the second step the second parameter of the gas parameters is determined as a function of the temperature measured values at the temperature sensor or at at least one further sensor of the temperature sensors.

2. Method according to Claim 1,
**characterized**
**in that** three independent gas parameters are determined as a function of the temperature measured values of the at least three temperature sensors, said gas parameters being determined by means of the computing device as a function of three linear independent linear combinations of the temperature measured values of the first, of the second and of the third temperature sensor.

3. Method according to Claim 1 or 2, **characterized**
**in that** at least one fourth temperature sensor measures the ambient temperature and/or the gas temperature outside the measuring range of the at least three temperature sensors and at a distance from the heating element, in particular before the gas enters the measuring range of the first temperature sensor and/or after it exits the measuring range of the third temperature sensor.

4. Method according to one of preceding claims, **characterized**
**in that** when at least two gas parameters are determined, the gas parameters are determined repeatedly, wherein for at least one of the gas parameters the determination is repeated with another time interval than for at least one other of the gas parameters.

5. Method according to one of the preceding claims, **characterized**
**in that** an uncorrected flow rate is determined as one of the gas parameters from the temperature measured values at the first and at the third temperature sensor, wherein a corrected flow rate is determined from the uncorrected flow rate and at least one further of the determined gas parameters.

6. Method according to one of the preceding claims, **characterized**
**in that** at least one of the determined gas parameters describes a thermal conductivity of the gas or a thermal diffusivity of the gas.

7. Method according to one of the preceding claims, **characterized**
**in that**, as one of the gas parameters, a corrected gas parameter which describes a thermal conductivity is determined as a function of an uncorrected gas parameter which describes a thermal conductivity and a gas parameter which describes a flow rate, and/or in that, as one of the gas parameters, a gas parameter which describes a temperature diffusivity is determined as a function of a gas parameter which describes a thermal conductivity, and a gas parameter which describes a flow rate is determined, said gas parameters being determined as a function of at least one instantaneous or previously acquired temperature measured value.

8. Method according to one of the preceding claims, **characterized**
**in that** for the determination of the gas parameter or at least one of the gas parameters an, in particular multi-dimensional, value table is used whose values are read out, in particular in interpolated form, as a function of the temperature measured values, respectively assigned to a dimension of the value table, of at least one of the temperature sensors.

9. Method according to one of the preceding claims, **characterized**
**in that** the temperature measured values of the temperature sensors do not unambiguously determine the gas parameter or at least one of the gas parameters, as a result of which given identical temperature measured values of all the temperature sensors a plurality of different values are determined for the gas parameter, wherein the selection of the specific value is dependent on preceding temperature measured values of at least one of the temperature sensors and/or preceding values of the same or of another of the gas parameters and/or known operating conditions of the flow meter, in particular information about expected gas compositions.

10. Method according to one of the preceding claims, **characterized**
**in that** for the calculation of the gas parameters the dependence of the gas parameter on the temperature values at the temperature sensors is approximated by linear or polynomial approximations.

11. Method according to one of the preceding claims, **characterized**
**in that** the gas parameter or one of the gas parameters is a mixture ratio of at least two gases, a concentration of a gas type or the gas type of a gas, and/or in that, in particular given known qualitative composition of the gas, a calorific value of the gas is determined as the gas parameter or at least one of the gas parameters.

12. Method according to one of the preceding claims, **characterized**
**in that** the heat output of the heating element is varied between a first value and at least one second value, in order to determine at least one further, independent gas parameter.

13. Method according to one of the preceding claims, **characterized**
**in that** a temperature measured value of at least one temperature sensor or a value derived therefrom is determined at a point in time at which the heating element is switched off, wherein at least one gas parameter is determined as a function of this temperature measured value.

14. Flow meter comprising a computing device (10) and a measuring section (2) with a heating element (4) and at least three temperature sensors (6, 7, 8) over which the gas is guided, wherein at least one first temperature sensor (6) is arranged upstream of the heating element (4), at least one second temperature sensor (7) is arranged in the region of the heating element (4), and at least one third temperature sensor (8) is arranged downstream of the heating element (4), and wherein the second temperature sensor (7) is formed, in particular, by the heating element (4),
**characterized**
**in that** the computing device (10) is designed to carry out the method according to one of Claims 1 to 13.

15. Flow meter according to Claim 14, **characterized**
**in that** said flow meter comprises a plurality of second temperature sensors (7) which are arranged upstream and downstream directly adjacent to the heating element (4) and provide a temperature measured value individually or jointly.

16. Flow meter according to Claim 14 or 15, **characterized in that**
said flow meter comprises an electronic switching unit which connects the first temperature sensor (6, 7, 8) or at least one of the first temperature sensors (6, 7, 8) to the second temperature sensor (6, 7, 8) or to at least one of the second temperature sensors (6, 7, 8) and/or to the third temperature sensor (6, 7, 8) or to at least one of the third temperature sensors (6, 7, 8) or connects the second temperature sensor (7, 8) or at least one of the second temperature sensors (7, 8) to the third temperature sensor (7, 8) or to at least one of the third temperature sensors (7, 8) in such a way that at least one output signal of the switching unit depends on the temperature measured values, combined in particular by addition or subtraction and/or multiplication and/or division, of the temperature sensors (6, 7, 8) which are connected by the switching unit, wherein the computing unit (10) is designed to determine the gas parameter or at least one of the gas parameters as a function of the output signal of the switching unit.

## Revendications

1. Procédé de détermination d'au moins un paramètre de gaz, en particulier d'un débit, d'un gaz en circulation, au moyen d'un débitmètre comprenant une section de mesure munie d'un élément chauffant et au moins trois capteurs de température sur lesquels le gaz est guidé, au moins un premier capteur de température étant disposé en amont de l'élément chauffant, au moins un deuxième capteur de température étant disposé dans la région de l'élément chauffant et au moins un troisième capteur de température étant disposé en aval de l'élément chauffant, en particulier l'élément chauffant lui-même pouvant être utilisé comme deuxième capteur de température, un dispositif de calcul déterminant l'au moins un paramètre de gaz en fonction des valeurs de mesure de température au niveau du premier, du deuxième et du troisième capteur de température, et/ou le dispositif de calcul déterminant au moins deux paramètres de gaz séparés en fonction des valeurs de mesure de température de différents capteurs de température et/ou des combinaisons de valeurs de mesure de température de différents capteurs de température, les valeurs de température sur le premier, deuxième et le troisième capteur de température étant utilisées pour déterminer les paramètres du gaz, **caractérisé en ce**
**qu'**au moins deux des paramètres du gaz sont déterminés dans un procédé itératif, alternativement dans une première étape au moins un premier des paramètres de gaz étant déterminé en fonction d'au moins un deuxième des paramètres de gaz et dans une deuxième étape au moins le deuxième des paramètres de gaz étant déterminé en fonction du premier des paramètres de gaz, dans la première étape le premier des paramètres de gaz étant déterminé en fonction de la valeur de mesure de température sur l'un au moins des capteurs de température et/ou dans la deuxième étape le deuxième des paramètres de gaz étant déterminé en fonction de la valeur de mesure de température sur le capteur de température ou sur au moins un autre des capteurs de température.

2. Procédé selon la revendication 1,
**caractérisé en ce que**
trois paramètres de gaz indépendants sont déterminés en fonction des valeurs de mesure de température des au moins trois capteurs de température et sont déterminés par le dispositif de calcul en fonction de trois combinaisons linéaires linéairement indépendantes des valeurs de mesure de température des premier, deuxième et troisième capteurs de température.

3. Procédé selon la revendication 1 ou 2,
**caractérisé en ce que**
la température ambiante et/ou la température du gaz est mesurée par au moins un quatrième capteur de température à l'extérieur de la zone de mesure des au moins trois capteurs de température et à distance de l'élément chauffant, en particulier avant l'entrée du gaz dans la zone de mesure du premier capteur de température et/ou après la sortie de la zone de mesure du troisième capteur de température.

4. Procédé selon l'une des revendications précédentes,
**caractérisé en ce que**
les paramètres de gaz sont déterminés de manière répétée lors de la détermination d'au moins deux paramètres de gaz, la détermination étant répétée, pour au moins un des paramètres de gaz, à un intervalle de temps différent de celui d'au moins un autre des paramètres de gaz.

5. Procédé selon l'une des revendications précédentes,
**caractérisé en ce que**
l'un des paramètres de gaz déterminé est un débit non corrigé parmi des valeurs de mesure de température au niveau des premier et troisième capteurs de température, un débit corrigé étant déterminé à partir du débit non corrigé et d'au moins un autre des paramètres de gaz déterminés.

6. Procédé selon l'une des revendications précédentes,
**caractérisé en ce**
**qu'**au moins un des paramètres de gaz déterminés décrit une conductivité calorifique du gaz ou une conductivité thermique du gaz.

7. Procédé selon l'une des revendications précédentes,
**caractérisé en ce que**
l'un des paramètres de gaz est un paramètre de gaz corrigé décrivant la conductivité calorifique qui est déterminé en fonction d'un paramètre de gaz non corrigé décrivant la conductivité calorifique et d'un paramètre de gaz décrivant un débit, et/ou l'un des paramètres de gaz est un paramètre de gaz décrivant la conductivité thermique qui est déterminé en fonction d'un paramètre de gaz décrivant la conductivité thermique et d'un paramètre de gaz décrivant un débit, lesquels sont déterminés en fonction d'au moins une valeur de mesure de température instantanée ou préalablement acquise.

8. Procédé selon l'une des revendications précédentes,
**caractérisé en ce**
**qu'**un tableau de valeurs, notamment multidimensionnel, est utilisé pour déterminer le paramètre de gaz ou au moins un des paramètres de gaz, tableau dont les valeurs sont lues, notamment interpolées, en fonction de la valeur de mesure de température, associée à une dimension du tableau de valeurs, d'au moins un des capteurs de température.

9. Procédé selon l'une des revendications précédentes,
**caractérisé en ce que**
les valeurs de mesure de température des capteurs de température ne déterminent pas de manière univoque le paramètre de gaz ou l'un au moins des paramètres de gaz de sorte que plusieurs valeurs différentes du paramètre de gaz sont déterminées pour des mêmes valeurs de mesure de température de tous les capteurs de température, le choix de la valeur déterminée dépendant de valeurs de mesure de température précédentes de l'un au moins des capteurs de température et/ou de valeurs précédentes du même paramètre de gaz, ou d'un autre, et/ou de conditions de fonctionnement connues du débitmètre, en particulier d'informations sur les compositions de gaz attendues.

10. Procédé selon l'une des revendications précédentes,
**caractérisé en ce que**
pour calculer le paramètre de gaz, la dépendance du paramètre de gaz vis-à-vis des valeurs de température au niveau des capteurs de température est approchée par des approximations linéaires ou polynomiales.

11. Procédé selon l'une des revendications précédentes,
**caractérisé en ce que**
le paramètre de gaz ou l'un des paramètres de gaz est un rapport de mélange d'au moins deux gaz, une concentration d'un type ou du type d'un gaz, et/ou **en ce que**, en particulier si la composition qualitative du gaz est connue, un pouvoir calorifique du gaz est déterminé comme le paramètre de gaz ou l'un au moins des paramètres de gaz.

12. Procédé selon l'une des revendications précédentes,
**caractérisé en ce que**
la puissance de chauffage de l'élément chauffant varie entre une première valeur et au moins une deuxième valeur afin de déterminer au moins un autre paramètre de gaz indépendant.

13. Procédé selon l'une des revendications précédentes,
**caractérisé en ce**
**qu'**une valeur de mesure de température d'au moins un capteur de température, ou une valeur qui en est dérivée, est déterminée à un instant où l'élément chauffant est désactivé, au moins un paramètre de gaz étant déterminé en fonction de cette valeur de mesure de température.

14. Débitmètre, comprenant un dispositif de calcul (10) et une section de mesure (2) munie d'un élément chauffant (4) et au moins trois capteurs de température (6, 7, 8) au-dessus desquels le gaz est guidé, au moins un premier capteur de température (6) étant disposé en amont de l'élément chauffant (4), au moins un deuxième capteur de température (7) étant disposé dans la région de l'élément chauffant (4) et au moins un troisième capteur de température (8) étant disposé en aval de l'élément chauffant (4), et le deuxième capteur de température (7) étant formé notamment par l'élément chauffant (4),
**caractérisé en ce que**
le dispositif de calcul (10) est conçu pour mettre en œuvre le procédé selon l'une des revendications 1 à 13.

15. Débitmètre selon la revendication 14,
**caractérisé en ce**
**qu'**il comprend une pluralité de deuxième capteurs de température (7) qui sont disposés en amont et en aval de manière directement adjacente à l'élément chauffant (4) et produisent individuellement ou conjointement une valeur de mesure de température.

16. Débitmètre selon la revendication 14 ou 15, **caractérisé en ce**
**qu'**il comprend une unité de circuit électronique qui relie le premier capteur de température ou l'un au moins des premiers capteurs de température au deuxième capteur de température ou à l'un au moins des deuxièmes capteurs de température et/ou au troisième capteur de température ou à l'un au moins des troisièmes capteurs de température (6, 7, 8) ou le deuxième capteur de température ou l'un au moins des deuxièmes capteurs de température au troisième capteur de température ou à l'un au moins des troisièmes capteurs de température (7, 8) de telle sorte qu'au moins un signal de sortie de l'unité de circuit dépende des valeurs de mesure de température combinées de manière linéaire, en particulier par addition ou soustraction, et/ou multiplication et/ou division des capteurs de température (6, 7, 8) reliés par l'unité de circuit, le dispositif de calcul (10) étant conçu pour déterminer le paramètre de gaz ou l'au moins un des paramètres de gaz en fonction du signal de sortie de l'unité de circuit.
